# EUROPÄISCHE PATENTSCHRIFT

(11) **EP 3 579 972 B1**
(45) Veröffentlichungstag und Bekanntmachung des Hinweises auf die Patenterteilung: **06.04.2022**
(21) Anmeldenummer: 18704941.6
(22) Anmeldetag: 06.02.2018
(51) Int. Cl.: B01L 3/00

(54) **MEDIZINALRÖHRCHEN**
MEDICAL TEST TUBE
TUBE MÉDICAL

(30) Priorität: 08.02.2017 CH 1452017
(43) Veröffentlichungstag der Anmeldung: 18.12.2019
(73) Patentinhaber: Roth, Felix, 9320 Arbon (CH)
(72) Erfinder: Roth, Felix, 9320 Arbon (CH)
(74) Vertreter: Keller Schneider Patent- und Markenanwälte AG
(86) Internationale Anmeldenummer: PCT/EP2018/052956
(87) Internationale Veröffentlichungsnummer: WO 2018/146093

(56) Entgegenhaltungen:
- EP-A2- 0 654 669
- WO-A1-2006/013599
- US-A- 3 977 598
- US-A1- 2013 045 852

## Beschreibung

### Technisches Gebiet

Die Erfindung betrifft ein Medizinalröhrchen zur Aufbereitung einer biologischen Probe mittels Dichtegradientenzentrifugation oder passiver Sedimentation.

### Technischer Hintergrund

Zentrifugieren ist eine gängige Methode um eine Probe zu fraktionieren. Häufig möchte man dabei eine Zielfraktion mit höherer Dichte vom umgebenden Medium abtrennen und diese Zielfraktion gleichzeitig reinigen und anreichern. Die sogenannte Dichtegradientenzentrifugation wird beispielsweise verwendet, um bewegliche Spermien von unbeweglichen zu trennen. Dabei können zudem unerwünschte Verunreinigungen wie Bakterien und Viren aus der Zielfraktion entfernt werden.

Um nach der Zentrifugation das Pellet (Niederschlag mit der Zielfraktion) vom Überstand zu trennen wird meist eine von zwei gängigen Methoden angewandt. Bei der ersten Methode wird der Überstand abgesaugt bis nur noch das Pellet mit der Zielfraktion übrigbleibt, welches anschliessend weiterverarbeitet wird. Bei der zweiten Methode wird eine Nadel durch den Überstand gestossen, um direkt an das Pellet zu gelangen und dieses abzusaugen. Bei beiden Methoden besteht jedoch die Gefahr, dass Verunreinigungen aus dem Überstand wieder in die Zielfraktion eingebracht werden. Dies ist beispielsweise beim Reinigen von Sperma von HIV-positiven Männern besonders problematisch, da so die Zielfraktion wieder mit HIV kontaminiert werden kann.

Um eine solche Rekontamination zu reduzieren schlägt DE60034720 ein Medizinalröhrchen zum Reinigen und Zentrifugieren von Sperma vor, welches am Boden einen stark verjüngten Abschnitt aufweist, in welchem nach der Zentrifugation das Pellet mit der Zielfraktion, resp. die gereinigten Spermien, enthalten ist. Der verjüngte Abschnitt wird dann an einer Sollbruchstelle vom übrigen Medizinalröhrchen abgetrennt, um die Zielfraktion anschliessend weiterzuverarbeiten. Beim Abtrennen des verjüngten Abschnitts besteht jedoch die Gefahr, dass der gesamte Überstand ausläuft. Zudem besteht beim Abtrennen der Zielfraktion eine erhöhte Gefahr des Einbringens von Verunreinigungen aus der unmittelbaren Umgebung.

Um eine Rekontamination zu reduzieren, beschreibt US2005064579 ein Medizinalröhrchen mit einem Einsatz, welcher in ein herkömmliches Medizinalröhrchen eingesetzt werden kann. Der Einsatz ist mittig mit einem Absaugröhrchen versehen, welches bis in den Bereich des Bodens des Medizinalröhrchens reicht, um nach der Zentrifugation die Zielfraktion resp. das Pellet abzusaugen. Nachteilig an dem Einsatz ist, dass beim Auftragen der Probe zwischen Absaugröhrchen und der Wandung des Medizinalröhrchens aufgrund der engen Platzverhältnisse die obere Öffnung des Absaugröhrchens kontaminiert werden kann, was bei Entnehmen der Zielfraktion wiederum zu einer Rekontamination führen kann.

EP2208540 beschreibt eine Medizinalröhrchen, bei welcher ein Einsatz eine Trennwand ausbildet, um einen Röhrcheninnenraum in zwei Kompartimente zu teilen. Bei allen Ausführungsformen sind die beiden Kompartimente zur Stirnseite des Röhrchen hin offen, um Proben aufzutragen und nach der Zentrifugation wieder zu entnehmen. Dazu muss ein Deckel die beiden Öffnungen abschliessender wieder entfernt werden und es besteht die Gefahr der Rekontamination. Um bei geschlossenem Deckel eine allfällig notwendiger Druckausgleich in den beiden Kompartimenten zu gewährleisten, können die beiden Kompartimente im oberen Bereich des Medizinalröhrchens mit Entlüftungsöffnungen versehen sein.

Aus EP1773496 ist ein abgedichtetes und steriles Medizinalröhrchen zum Reinigen einer biologischen Probe bekannt, dessen Deckel mit einer Probeentnahmeöffnung und einer Einfüllöffnung versehen ist. Die Probeentnahmeöffnung ist mit einer Nadel versehen, welche zur Probeentnahme bis in den Bereich des Bodens des Medizinalröhrchens reicht. Die Einfüllöffnung ist mit einem durchstechbaren Septum versehen. Um bei geschlossenem Deckel das Einfüllen und Entnehmen der Probe zu ermöglichen, ist der Deckel mit einer einen Filter aufweisenden Druckausgleichsöffnung versehen. Der Deckel ist relativ komplex im Aufbau und benötigt viele Montageschritte, was die Herstellungskosten erheblich erhöht. Ein weiterer Nachteil besteht darin, dass bei einer Dichtegradientenzentrifugation die Medien zum Aufbau des Dichtegradienten durch die Einfüllöffnung bei aufgesetztem Deckel eingefügt werden müssen, da ansonsten beim Verschliessen des Deckels der Dichtegradient aufgebwirbelt werden kann. Ähnliche Medizinalröhrchen sind aus WO2005039773, US7717274 und WO2002098566 bekannt.

GB1064901 beschreibt ein Röhrchen, welches in der Seitenwand einen Kanal aufweist, welcher vom tiefsten Punkt am Röhrchenboden bis nach oben in die Stirnfläche geführt ist. Auch bei diesem Röhrchen besteht die Gefahr einer Rekontamination.

US2013045852 offenbart ein Zentrifugationsgefäß mit einer Außenwand, die einen Innenraum enthält, wobei ein Damm eine Barriere definiert, die den Innenraum in mindestens zwei Bereiche teilt. Ein Auffangbecken umfasst ein Entnahmerohr, das sich vom Deckel nach unten erstreckt und mit einer Auslassöffnung in Verbindung steht.

### Darstellung der Erfindung

Eine Aufgabe der Erfindung ist es, die obigen Probleme zu vermeiden und insbesondere eine Rekontamination effizient zu verhindern. Das Medizinalröhrchen soll zudem einfach in der Verwendung und Herstellung sein. Diese Aufgabe wird durch ein Medizinalröhrchen mit den Merkmalen des Anspruchs 1 gelöst. Das Medizinalröhrchen zur Aufbereitung einer biologischen Probe mittels Dichtegradientenzentrifugation und/oder passiver Sedimentation umfasst einen röhrchenförmigen Behälter mit einer im Wesentlichen zylindrischen Behälterwand, einer oberen Einfüllöffnung und einem unteren Behälterboden, und einen die obere Einfüllöffnung abdeckenden Behälterdeckel, insbesondere ein Schraubdeckel. Die Behälterwand umschliesst einen Behälterinnenraum. Im Behälter ist ein Probeentnahmekanal angeordnet ist, welcher eine obere Probeentnahmeöffnung und eine untere Probeentnahmeöffnung aufweist, wobei der Probeentnahmekanal durch die untere Probeentnahmeöffnung im Bereich des Behälterbodens in den Behälterinnenraum mündet. Abgesehen von der unteren Probeentnahmeöffnung ist der Probeentnahmekanal zum Behälterinnenraum abgeschlossen. Die obere Probeentnahmeöffnung ist zur Entnahme einer Probe mittels eines Probeentnahmeschlauchs seitlich im oberen Bereich in der Behälterwand angeordnet, so dass die Probe bei geschlossenem Behälterdeckel durch die obere Probeentnahmeöffnung entnehmbar ist. Ein an die Probeentnahmeöffnung anschliessender Umlenkbereich des Probeentnahmekanals weist zur leichteren Einführung des Probeentnahmeschlauches eine gerundete, obere Umlenkfläche auf, so dass der Probeentnahmekanal in Richtung Einfüllöffnung geschlossen ausgebildet ist. Bevorzugt ist die Probeentnahmeöffnung im oberen Bereich des Medizinalröhrchens, vorzugsweise im oberen Viertel der Behälterwand oder direkt unterhalb des Behälterdeckels, angeordnet.

Ein solches Medizinalröhrchen oder Zentrifugationsröhrchen kann also einen herkömmlichen Schraubdeckel ohne irgendwelche Öffnungen aufweisen. Die Medien für die verschiedenen Lagen des Dichtegradienten und anschliessend die Probe, z.B. eine Spermienprobe, können bei entferntem Behälterdeckel durch die Einfüllöffnung des Medizinalröhrchen eingefüllt werden. Ein Kontamination des Probeentnahmekanals ist dabei nicht möglich. Anschliessend wird das Medizinalröhrchen mit dem Deckel verschlossen und zentrifugiert. Nach erfolgter Zentrifugation kann das Pellet mit der gereinigten Zielfraktion vom Behälterboden des Medizinalröhrchens entnommen werden, indem ein dünner Entnahmeschlauch seitlich durch die Probeentnahmeöffnung in den Probeentnahmekanal zum Pellet geführt wird. Eine Rekontamination der Zielfraktion mit in den oberen Lagen des Dichtegradienten verbleibenden Verunreinigungen wird verhindert, da der Entnahmeschlauch aufgrund des vom Innenraum abgeschlossenen Probeentnahmekanals mit diesen nicht in Berührung kommt. Zudem wird die Gefahr einer allfälligen Kontamination der Umgebung (z.B. bei HIV-kontaminierten Proben) verringert, da der Röhrchendeckel für den Entnahmevorgang nicht mehr geöffnet werden muss.

In einigen Ausführungsformen kann der Behälter ein die obere Probeentnahmeöffnung abdeckendes, temporäres Verschlusselement aufweisen. Diese kann nach erfolgter Zentrifugation resp. vor dem Entnehmen der Probe entfernt werden. Bevorzugt schliesst das Verschlusselement die Probeentnahmeöffnung nicht gasdicht ab, so dass ein Druckausgleich im Probenentnahmekanal jederzeit möglich ist. So kann beispielsweise auch der Behälterdeckel und der obere Behälterrand derart ausgebildet sein, dass ein Druckausgleich des Behälterinnenraums möglich ist. Beispielsweise kann der Behälterrand dazu einen dünnen resp. feinen Einschnitt oder eine Druckausgleichskerbe aufweisen. Der Einschnitt resp. die Kerbe ist in der Regel derart schmal ausgestaltet, dass sie zwar luftdurchlässig ist, aber z.B. bei einem Umfallen des Röhrchens aufgrund der Kapillarwirkung flüssigkeitsundurchlässig ist.

In einigen Ausführungsformen kann das Verschlusselement ein Öffnungsschieber zum Öffnen und Verschliessen der oberen Probeentnahmeöffnung sein. Der Öffnungsschieber kann als eine den Behälter wenigstens teilweise umgreifende Hülse ausgebildet sein, welche eine Öffnung aufweist, die zum Öffnen der Probeentnahmeöffnung mit dieser in Überdeckung bringbar ist, z.B. durch Drehung um eine Längsachse des Medizinalröhrchen. Um den Öffnungsschieber einfach von einer Geschlossenstellung in eine Offenstellung zu bewegen, kann der Öffnungsschieber und der Behälter mit einem Anschlag versehen sein, die bei vollständig geöffnetem Öffnungsschieber in Anschlag miteinander sind.

In einigen Ausführungsformen kann der Öffnungsschieber und der Behälter Rastmittel aufweisen, welche den Öffnungsschieber in einer Offenstellung und in einer Geschlossenstellung einrasten.

In einigen Ausführungsformen kann das Verschlusselement eine die Probeentnahmeöffnung abdeckende Abreisshülse sein, welche zum Öffnen der Probeentnahmeöffnung wenigstens teilsweise abreissbar ist und entlang einer Reissnaht Sollbruchstellen oder Sollreissstellen aufweist.

In einigen Ausführungsformen kann das Verschlusselement eine die Probeentnahmöffnung abdeckende Dünnstelle aufweisen, welche mittels eines Probeentnahmemittels durchstochen werden kann.

In einigen Ausführungsformen kann das Verschlusselement in die Behälterwand eingelassen sein, so dass Aussenflächen der Behälterwand und des Verschlusselements wenigstens unterhalb des Verschlusselements miteinander fluchten.

Das Medizinalröhrchen ist bevorzugt ein mittels Spritzgiessen aus Kunststoff hergestellter mehrteiliger Spritzgussartikel sein. Das Verschlusselement kann dabei ein separates Spritzgussteil sein, z.B. im Falle eines Öffnungsschiebers oder im Falle eines über Sollreissstellen verbundenes Verschlusselement, oder ein am Behälterdeckel oder am oberen Behälterteil integral angeformtes Teil darstellen, z.B. im Falle eines über Sollreissstellen verbundenes Verschlusselement oder eines Verschlusselements in Form einer Dünnstelle.

In einigen Ausführungsformen kann der Behälter zweiteilig aus einem oberen Behälterteil und einem unteren Behälterteil ausgebildet ist, wobei der obere Behälterteil und der untere Behälterteil miteinander in Eingriff bringbar sind und der obere Behälterteil die obere Probeentnahmeöffnung und die obere Umlenkfläche ausbildet. Der obere Behälterteil und der untere Behälterteil können mittels umlaufenden Rastmitteln, insbesondere einer umlaufende Wulst und einer umlaufende Nut, miteinander in Eingriff bringbar sein.

In einigen Ausführungsformen kann zwischen dem oberen Behälterteil und dem unteren Behälterteil eine Verdrehsicherung angeordnet sein, vorzugsweise in Form mindestens einer Aussparung und einem dazu komplementären Vorsprung.

In einigen Ausführungsformen kann der obere Behälterteil den Probeentnahmekanal ausbilden, welcher in den Behälterinnenraum hineinreicht. Der Probeentnahmekanal kann als im Behälterinnenraum freistehendes, dünnen Rohr oder Röhrchen ausgebildet sein, welches mit dem oberen Behälterteil verbunden ist und bis zum Behälterboden hinabragt. Der Probeentnahmekanal kann also am oberen Behälterteil angeformt sein oder der Probeentnahmekanal kann zweiteilig ausgebildet sein, wobei ein oberer, erster Kanalteil durch den oberen Behälterteil ausgebildet ist und ein unterer, zweiter Kanalteil durch ein in den Behälterinnenraum hineinreichendes Röhrchen ausgebildet ist, welches fest im oberen Behälterteil gehalten ist.

Bevorzugt schliessen die Innenflächen des oberen ersten Kanalteils im Übergangsbereich zum unteren, zweiten Kanalteil mit den Innenflächen des unteren, zweiten Kanalteils bündig ab, so dass Probeentnahmemittel z.B. eine Probeentnahmeschlauch, beim Einführen nicht hängen bleiben. D.h. der untere, zweite Kanalteil könnte im Übergangsbereich auch einen grösseren Innenquerschnitt als der obere, erste Kanalteil aufweisen.

Das Medizinalröhrchen kann mehrteilig aus Kunststoff einfach mittels Spritzgussverfahren hergestellt werden.

In einigen Ausführungsformen kann die Probeentnahmeöffnung im oberen Bereich des Medizinalröhrchens, vorzugsweise im oberen Drittel der Röhrchenwand, unterhalb des Deckels angeordnet sein. Dabei ist in der Regel die Probeentnahmeöffnung nicht durch den Deckel abgedeckt. Es ist jedoch auch möglich, dass der Behälterdeckel mit einem sich nach unten erstreckenden Verschlusselement z.B. in Form einer Hülse versehen ist, die über die Probeentnahmeöffnung reicht und diese abdeckt. Um das Pellet nach dem Zentrifugieren zu entnehmen, kann der Behälterdeckel entfernt werden, damit die Probeentnahmeöffnung freigegeben wird. Bevorzugt kann das Verschlusselement auch über eine Sollbruchstelle mit dem Deckel verbunden sein, so dass es ohne den Deckel abnehmen zum müssen vom Medizinalröhrchen entfernt werden kann.

Das Medizinalröhrchen kann als Wegwerf-Medizinalröhrchen aus Kunststoff gefertigt sein.

Die Erfindung betrifft weiter die Verwendung eines voran beschriebenen Medizinalröhrchens zur Selektion und Reinigung von Spermien mittels Dichtegradientenzentrifugation und/oder passiver Sedimentation.

Ein Reinigungsverfahren einer biologischen Probe, z.B. Spermien, kann dabei folgende Schritte enthalten:
- Bereitstellen eines sterilen voran beschriebenen Medizinalröhrchens;
- Einfüllen und Aufbauen eines Dichtegradienten im Innenraum des Medizinalröhrchen durch die Einfüllöffnung;
- Auftragen der biologischen Probe durch die Einfüllöffnung auf die oberste Lage des Dichtegradientens und Aufsetzen des Behälterdeckels;
- Zentrifugieren des Medizinalröhrchens mit der geladenen Probe bis sich am Behälterboden das gewünschte Pellet mit der Zielfraktion bildet;
- Entfernen oder Öffnen des Verschlusselements zum Öffnen der Probeentnahmeöffnung;
- Einführen eines Probeentnahmeschlauchs in den Probeentnahmekanal bis zum Pellet hin bei aufgesetztem Behälterdeckel;
- Entnehmen des Pellets durch den Probenentnahmeschlauch; und
- Entsorgung des Medizinalröhrchens.

### Kurze Erläuterung zu den Figuren

Die Erfindung soll nachfolgend anhand von Ausführungsbeispielen im Zusammenhang mit der(n) Zeichnung(en) näher erläutert werden. Es zeigen:
- Fig. 1: eine Schnittdarstellung eines ersten Ausführungsbeispiels eines Medizinalröhrchens (ohne Deckel);
- Fig. 2: ein oberer und ein unterer Behälterteil des Ausführungsbeispiels aus Fig. 1 in einer perspektivischen Explosionsdarstellung;
- Fig. 3: ein oberer Behälterteil des Ausführungsbeispiels aus Fig. 1 in einer perspektivischen Ansicht von unten;
- Fig. 4: eine perspektivische Ansicht eines zweiten Ausführungsbeispiels eines Medizinalröhrchens (ohne Deckel);
- Fig. 5: eine Schnittdarstellung des oberen Bereichs des Ausführungsbeispiels aus Fig. 4;
- Fig. 6: zwei perspektivische Ansichten des oberen Behälterteils des Ausführungsbeispiels aus Fig. 4;
- Fig. 7: eine Explosionsdarstellung eines dritten Ausführungsbeispiels eines Medizinalröhrchens mit Öffnungsschieber;
- Fig. 8: eine Schnittdarstellung des Ausführungsbeispiels aus Fig. 7;
- Fig. 9: perspektivische Ansichten des oberen Bereichs des Ausführungsbeispiels aus Fig. 7 unter (a) in einer Offenstellung und unter (b) in Geschlossenstellung; und

- Fig. 10: perspektivische Ansicht des oberen Behälterteils mit Öffnungsschieber.

### Wege zur Ausführung der Erfindung

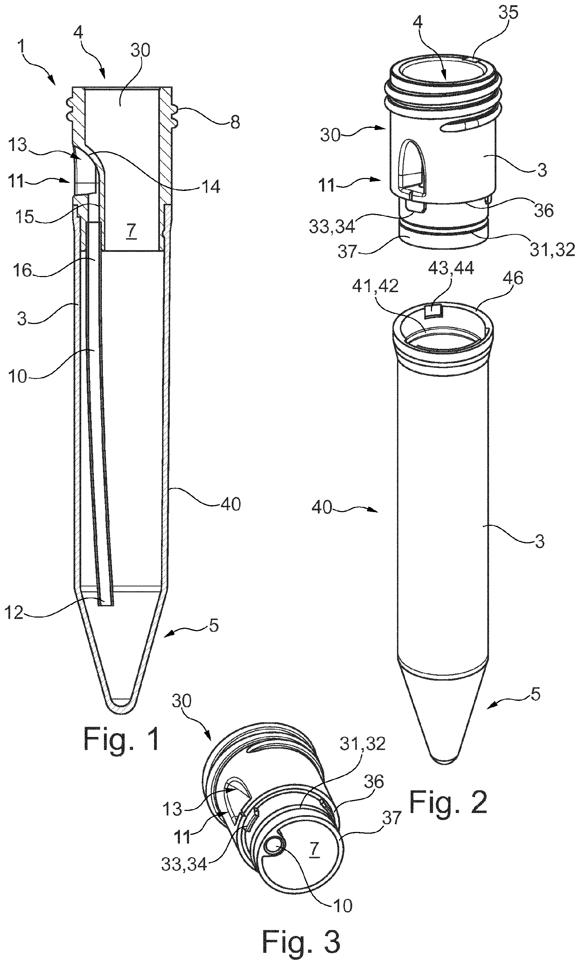

Die Figuren 1 bis 3 zeigen ein erstes Ausführungsbeispiel eines Medizinalröhrchens (ohne Deckel) zur Aufbereitung einer biologischen Probe mittels Dichtegradientenzentrifugation und/oder passiver Sedimentation. Fig. 1 zeigt das Medizinalröhrchen in einer Schnittdarstellung. Fig. 2 zeigt einen oberen Behälterteil 30 und einen unteren Behälterteil 40 in einer perspektivischen Explosionsdarstellung. Fig. 3 zeigt den oberen Behälterteil 30 in einer perspektivischen Ansicht von unten.

Das Medizinalröhrchens bildet einen im Wesentlichen zylindrischen oder röhrchenförmigen Behälter 1 mit einer einen Behälterinnenraum 7 umschliessende, dünnwandige Behälterwand 3. Oben ist eine Einfüllöffnung 4 ausgebildet. Unten ist der der Behälter 1 mit einem konisch zulaufenden Behälterboden 5 abgeschlossen. Die Einfüllöffnung 4 ist mittels eines Behälterdeckels 6, vorzugsweise ein Schraubdeckel, verschliessbar. Im gezeigten Ausführungsbeispiel ist um die Einfüllöffnung 4 eine Aussengewinde 8 für einen Schraubdeckel ausgebildet. Bevorzugt entspricht die äussere Kontur einem standardmässigen 15 ml oder 50 ml Laborröhrchen (auch unter dem Namen "BD Falcon^{™}-Tubes" bekannt) und passt entsprechend in die herkömmlichen Zentrifugen.

Im Behälterinnenraum 7 ist eine Probeentnahmekanal 10 ausgebildet. Der Probeentnahmekanal 10 ist vom Behälterinnenraum 7 abgegrenzt und weist lediglich am unteren Ende eine untere Probeentnahmeöffnung 12 auf, welche im Bereich des Behälterbodens 5 in den Innenraum 7 des Medizinalröhrchens mündet. Am oberen Ende weist der Probeentnahmekanal 10 eine obere Probeentnahmeöffnung 11 auf, welche seitlich in der Behälterwand 3 angeordnet ist und über welche der Probeentnahmekanal 10 in die Umgebung ausserhalb des Medizinalröhrchens mündet. Der Probeentnahmekanal 10 dient zur Entnahme einer Probe vom Behälterboden 5 nach der Zentrifugation. Dazu kann ein dünner Probeentnahmeschlauch bis zur pelletierten Probe geführt werden, ohne das über dem Pellet liegende Medium zu kontaktieren oder zu verwirbeln. Indem der Probeentnahmekanal 10 über die obere Probeentnahmeöffnung 11 seitlich in der Behälterwand 3 mündet, kann eine Probe vom Behälterboden 6 entnommen werden, ohne den Behälterdeckel 4 entfernen zu müssen. Allfällige Rekontaminationen der Probe werden dadurch verhindert.

Der Probeentnahmekanal 10 ist derart dimensioniert, dass ein dünner Probeentnahmeschlauch von 1 mm bis 2.5 mm durch den Probeentnahmekanal 10 bis zum Röhrchenboden 6 resp. bis zum Pellet eingeführt werden kann. D.h. der Innendurchmesser liegt bevorzugt zwischen 1.5 mm und 4 mm.

Zur erleichterten Einführung eines Probeentnahmeschlauches in den Probeentnahmekanal 10 weist der Probeentnahmekanal 10 am oberen Ende einen Umlenkbereich 13 auf. In diesem Umlenkbereich 13 ist eine gerundete Umlenkfläche 14 derart ausgebildet, dass ein seitlich eingeführter Probeentnahmeschlauch an der Umlenkfläche 14 nach unten in Richtung Behälterboden umgelenkt und in den Probeentnahmekanal 10 geführt wird. Die obere Probenentnahmeöffnung 11 kann als längliches Loch in der Form eines Torbogens ausgebildet sein.

Das Medizinalröhrchen der Figuren 1 bis 3 weist einen zweiteiligen Behälter 1 mit einem oberen Behälterteil 30 und einem unteren Behälterteil 40 auf. Die beiden Behälterteile 30, 40 sind verdrehsicher ineinander gesteckt. Die Verdrehsicherung 33, 43 wird mittels in komplementäre Kerben 44 passende Vorsprünge 34 ausgebildet, wobei in der gezeigten Ausführungsform die Kerben 44 innenseitig im oberen Rand 46 des unteren Behälterteils 40 ausgebildet sind. Die komplementären Vorsprünge 34 sind am oberen Behälterteil 30 unterhalb einer umlaufenden Schulter 36 an einer sich nach unten erstreckenden Schürze 37 ausgebildet. Der obere Behälterteil 30 wird mit der Schürze 37 in den unteren Behälterteil 40 gesteckt. Um in darin festzuhalten weist der obere Behälterteil 30 in der Schürze 37 ein Einrastmittel 31 in Form einer umlaufenden Nut 32 auf. Der untere Behälterteil 40 weist an der Innenseite ein komplementäres Einrastmittel 41 in Form einer umlaufenden Wulst 42 auf. Im zusammengesetzten Zustand des oberen und unteren Behälterteils liegt die Schulter 36 des oberen Behälterteils auf einem oberen Rand 46 des unteren Behälterteils auf.

Der Probeentnahmekanal 10 ist zweiteilig ausgebildet mit einem oberen, ersten Kanalteil 15 und einem unteren, zweiten Kanalteil 16. Der obere, erste Kanalteil 15 ist im oberen Behälterteil 30 integriert und bildet auch den Umlenkbereich 13 mit der Umlenkfläche 14 auf. Der untere, zweite Kanalteil 16 (in Fig. 2 und 3 nicht dargestellt) ist ein schmales Röhrchen, welches in einer Öffnung des oberen Kanalteils 15 befestigt ist. Bevorzugt schliessen die Innenflächen des oberen ersten Kanalteils 15 im Übergangsbereich zum unteren, zweiten Kanalteil 16 mit den Innenflächen des unteren, zweiten Kanalteils 16 bündig ab, so dass Probeentnahmemittel z.B. eine Probeentnahmeschlauch, beim Einführen nicht hängen bleiben. D.h. der untere, zweite Kanalteil 16 könnte im Übergangsbereich auch einen grösseren Innenquerschnitt als der obere, erste Kanalteil 15 aufweisen.

Bei der Zentrifugation oder bei der Probeentnahme können im Behälterinnenraum 7 Druckunterschiede zur Umgebung entstehen. Diese können auch bei geschlossenenm Deckel über eine Druckausgleichskerbe 35 im oberen Rand des Behälters ausgeglichen werden. Diese kann derart schmal ausgestaltet sein, dass sie zwar luftdurchlässig ist, aber z.B. bei einem Umfallen des Röhrchens aufgrund der Kapillarwirkung flüssigkeitsundurchlässig ist.

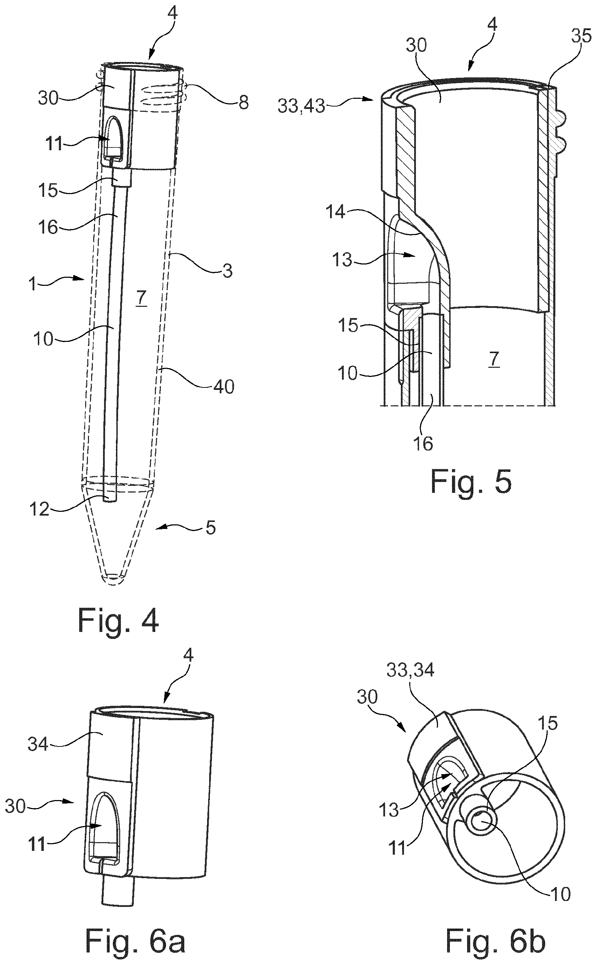

In den Figuren 4 bis 6 ist ein zweites Ausführungsbeispiel eines Medizinalröhrchens (ohne Deckel) gezeigt. Fig. 4 zeigt das Medizinalröhrchen in einer perspektivischen Darstellung mit durchsichtigem unteren Behälterteil. Fig. 5 zeigt eine Schnittdarstellung des oberen Bereichs des Medizinalröhrchens. Fig. 6 zeigt zwei perspektivische Ansichten des oberen Behälterteils.

Im Unterschied zum Ausführungsbeispiel der Fig. 1 ist insbesondere die Verdrehsicherung anders ausgestaltet. Das obere Behälterteil 30 mit der oberen Probeentnahmeöffnung 11 ist grösstenteils in den unteren Behälterteil 40 eingeschoben und bildet zusammen mit diesem den oberen Rand des Behälters 1. Im Bereich der oberen Probeentnahmeöffnung 11 ist es radial nach aussen vorgesetzt und bildet einen Vorsprung 34 der Verdrehsicherung aus, welcher formschlüssig in eine komplementäre Kerbe oder in etwa rechteckige Aussparung des unteren Behälterteils 40 passt. Das Aussengewinde 8 für den Schraubdeckel ist am unteren Behälterteil 40 ausgebildet.

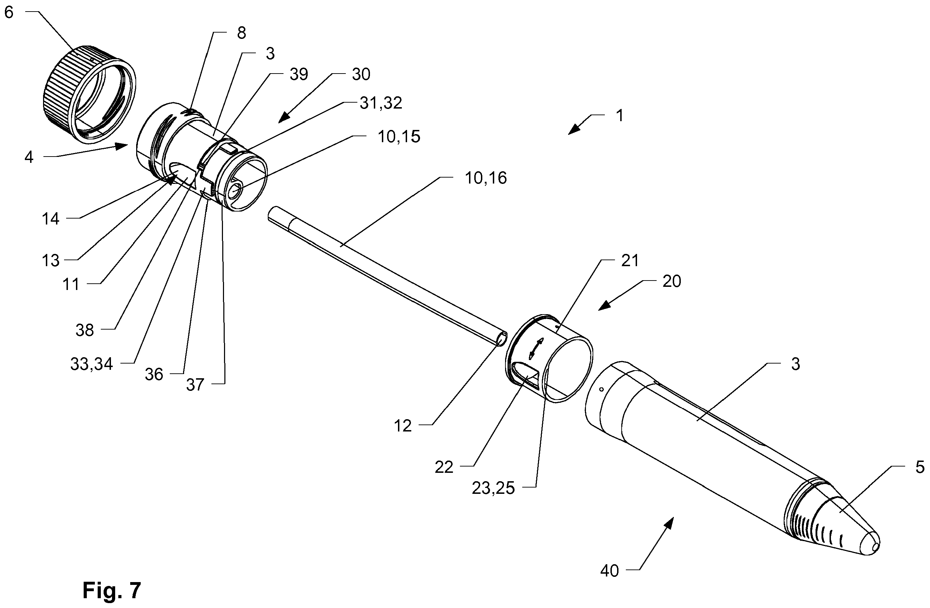

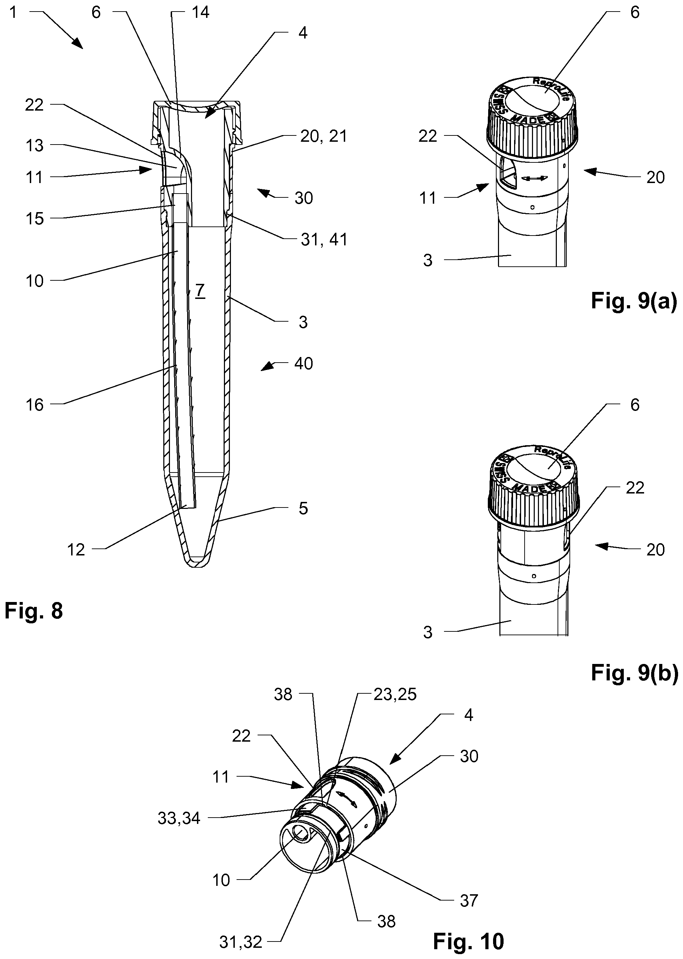

Die Figuren 7 bis 10 zeigen eine dritte Ausführungsform eines Medizinalröhrchens mit Behälterdeckel 6. Fig. 7 zeigt eine Explosionsdarstellung. Fig. 8 zeigt eine Schnittdarstellung. Fig. 9 zeigt eine einen Ausschnitt des Medizinalröhrchens mit einem Öffnungschieber 20 unter (a) in einer Offenstellung und unter (b) in einer Geschlossenstellung. Fig. 10 zeigt eine perspektivische Ansicht des oberen Behälterteils mit Öffnungsschieber.

Wie das Ausführungsbeispiel aus Fig. 1 weist das Medizinalröhrchen der Fig. 7 einen Behälterdeckel 6, eine oberen Behälterteil 30, welcher den oberen, ersten Kanalteil 15 mit der oberen Probeentnahmeöffnung 11 ausbildet, einen unteren zweiten Kanalteil 16 in Form eine schmalen Röhrchens und einen unteren Behälterteil 40 auf. Im Unterschied zum Ausführungsbeispiel aus Fig. 1 weist das Ausführungsbeispiel aus Fig. 7 eine zusätzliches Verschlusselement 20 in Form eines Öffnungsschiebers zum Öffnen und Schliessen der oberen Probeentnahmeöffnung 11 auf.

Der Öffnungsschieber 20 ist im Wesentlichen als Hülse 21 ausgestaltet und umgreift den oberen Behälterteil 30, der in diesem Bereich entsprechen verjüngt ausgestaltet ist. Die äussere Oberfläche der Hülse 21 und die äussere Oberfläche des unteren Behälterteils 40 fluchten miteinander.

Der Öffnungschieber 20 weist eine Öffnung 22, deren Dimension sich mit der Dimension der oberen Probeentnahmeöffnung 11 deckt. Durch Drehung des Öffnungsschiebers 20 um die Längsachse des Medizinalröhrchens, ist die Öffnung 22 des Öffnungsschiebers 20 mit der Probeentnahmeöffnung 11 in Übereinstimmung bringbar, um nach erfolgter Zentrifugation die Probe zu entnhemen, ohne dass der Behälterdeckel 6 entfernt werden muss. Fig 9(a) zeigt dabei die Offenstellung des Medizinalröhrchen, in welcher die beiden Öffnungen in Übereinstimmung sind. Fig. 9(b) zeigt die Geschlossenstellung.

Behälter 1 resp. oberes Behälterteil 30 und Öffnungsschieber 20 können zur vereinfachten Bedienung jeweils einen Anschlag 23, 38 aufweisen, welcher die Offen- resp. Geschlossenstellung definiert. In den beiden Stellungen kann der Öffnungschieber mittels Rastmitteln 25, 39 einrasten. Im gezeigten Ausführungsbeispiel sind die Rastmittel am Öffnungschieber als Vorsprung 25 und am Behälter als korrspondierende Kerbe 39 ausgestaltet.

Die vorangehend beschriebenen Medizinalröhrchen mit seitlich in der Behälterwand sich öffnenden Probeentnahmekanal können für die Dichtegradientenzentrifugation einer biologischen Probe, z.B. zur Reinigung und Trennung einer Spermienprobe, verwendet werden. Dazu wird zuerst der Behälterdeckel entfernt und mehrere Lagen eines Dichtegradienten werden im Behälterinnenraum des Medizinalröhrchen aufgebaut. Anschliessend wird die Probe eingefüllt, der Behälterdeckel aufgesetzt und das Medizinalröhrchen mit Probe ist bereit für die Zentrifugation. Nach erfolgter Zentrifugation befindet sich die Zielfraktion der Probe im Pellet am Behälterboden des Medizinalröhrchens und kann direkt vom Behälterboden entnommen werden. Dazu wird die Probeentnahmeöffnung geöffnet und ein Probeentnahmeschlauch kann bei geschlossenem Behälterdeckel durch den Probeentnahmekanal bis zum Pellet am Behälterboden geführt werden. Der Probeentnahmeschlauch kommt dabei nicht mit allfälligen Kontaminanten aus den oberen Lagen des Dichtegradienten und dem Überstand der Probe in Kontakt und eine Rekontamination ist effizient verhindert. Das Pellet mit der Zielfraktion kann z.B. mittels einer Spritze durch den Probenentnahmeschlauch abgesaugt werden. Analog kann das Medizinalröhrchen für allfällig folgende Waschschritte verwendet werden. Dazu wird die entnommene Zielfraktion in ein neues Röhrchen überführt, mit einer Waschlösung versehen und nach dem Zentrifugieren oder passivem Sedimentieren auf die oben beschriebene Weise die Zielfraktion erneut entnommen.

Die Ausgestaltung des oberen Probeentnahmekanals mit dem Umlenkbereich, die Zweiteiligkeit des Behälters oder der Öffnungsschieber können zusammen mit dem seitlich offenen Probeentnahmekanal als eigenständige Erfindungen betrachtet werden.

### Bezeichnungsliste

- 1: röhrchenförmiger Behälter
- 3: Behälterwand
- 4: Einfüllöffnung
- 5: Behälterboden
- 6: Behälterdeckel
- 7: Behälterinnenraum
- 8: Aussengewinde
- 10: Probeentnahmekanal
- 11: obere Probeentnahmeöffnung
- 12: untere Probeentnahmeöffnung
- 13: Umlenkbereich
- 14: Umlenkfläche
- 15: erster Kanalteil
- 16: zweiter Kanalteil
- 20: Verschlusselement / Öffnungsschieber
- 21: Hülse
- 22: Öffnung
- 23: Anschlag
- 25: Rastmittel, Vorsprung
- 30: oberen Behälterteil
- 31: Rastmittel
- 32: Nut
- 33: Verdrehsicherung
- 34: Vorsprung
- 35: Druckausgleichskerbe
- 36: Schulter
- 37: Schürze
- 38: Anschlag
- 39: Rastmittel, Kerbe
- 40: unteren Behälterteil
- 41: Rastmittel
- 42: Wulst
- 43: Verdrehsicherung
- 44: Aussparung

## Patentansprüche

1. Medizinalröhrchen zur Aufbereitung einer biologischen Probe mittels Dichtegradientenzentrifugation und/oder passiver Sedimentation umfassend einen röhrchenförmigen Behälter (1) mit einer im Wesentlichen zylindrischen Behälterwand (3), einer oberen Einfüllöffnung (4) und einem unteren Behälterboden (5), und einen die obere Einfüllöffnung (4) abdeckenden Behälterdeckel (6), insbesondere ein Schraubdeckel; wobei die Behälterwand (3) einen Behälterinnenraum (7) umschliesst; wobei im Behälter (1) ein Probeentnahmekanal (10) angeordnet ist, welcher eine obere Probeentnahmeöffnung (11) und eine untere Probeentnahmeöffnung (12) aufweist, wobei der Probeentnahmekanal (10) durch die untere Probeentnahmeöffnung (12) im Bereich des Behälterbodens (5) in den Behälterinnenraum (7) mündet, **dadurch gekennzeichnet, dass** die obere Probeentnahmeöffnung (11) zur Entnahme einer Probe mittels eines Probeentnahmeschlauchs seitlich im oberen Bereich in der Behälterwand (3) angeordnet ist, so dass die Probe bei geschlossenem Behälterdeckel (6) durch die obere Probeentnahmeöffnung (11) entnehmbar ist, wobei ein an die Probeentnahmeöffnung (11) anschliessender Umlenkbereich (13) des Probeentnahmekanals (10) zur leichteren Einführung des Probeentnahmeschlauches eine gerundete, obere Umlenkfläche (14) aufweist.

2. Medizinalröhrchen nach Anspruch 1, **dadurch gekennzeichnet, dass** der Behälter ein die obere Probeentnahmeöffnung (11) abdeckendes Verschlusselement (20) aufweist.

3. Medizinalröhrchen nach Anspruch 2, **dadurch gekennzeichnet, dass** das Verschlusselement ein Öffnungsschieber (20) zum Öffnen und Verschliessen der oberen Probeentnahmeöffnung (11) ist.

4. Medizinalröhrchen nach Anspruch 3, **dadurch gekennzeichnet, dass** der Öffnungsschieber (20) eine den Behälter (1) umgreifende Hülse (21) ist, welche eine Öffnung (22) aufweist, die zum Öffnen der oberen Probeentnahmeöffnung (11) mit dieser in Überdeckung bringbar ist.

5. Medizinalröhrchen nach einem der Ansprüche 3 oder 4, **dadurch gekennzeichnet, dass** der Öffnungsschieber (20) und der Behälter (1) jeweils mit einem Anschlag (23, 38) versehen sind, die bei vollständig geöffnetem Öffnungsschieber in Anschlag miteinander sind.

6. Medizinalröhrchen nach einem der Ansprüche 3 bis 5, **dadurch gekennzeichnet, dass** der Öffnungsschieber (20) und der Behälter (1) Rastmittel (25, 39) aufweisen, welche den Öffnungsschieber in einer Offenstellung und in einer Geschlossenstellung einrasten.

7. Medizinalröhrchen nach Anspruch 2, **dadurch gekennzeichnet, dass** das Verschlusselement eine die Probeentnahmeöffnung abdeckende Abreisshülse ist, welche zum Öffnen der Probeentnahmeöffnung wenigstens teilsweise abreissbar ist und entlang einer Reissnaht Sollbruchstellen oder Sollreissstellen aufweist.

8. Medizinalröhrchen nach einem der Ansprüche 2 bis 6, **dadurch gekennzeichnet, dass** das Verschlusselement (20) in die Behälterwand (2) eingelassen ist, so dass Aussenflächen der Behälterwand (2) und des Verschlusselements (20) wenigstens unterhalb des Verschlusselements miteinander fluchten.

9. Medizinalröhrchen nach einem der vorangehenden Ansprüche, **dadurch gekennzeichnet, dass** der Behälter (1) zweiteilig aus einem oberen Behälterteil (30) und einem unteren Behälterteil (40) ausgebildet ist, wobei der obere Behälterteil (30) und der untere Behälterteil (40) miteinander in Eingriff bringbar sind und der obere Behälterteil (30) die obere Probeentnahmeöffnung (11) und die obere Umlenkfläche (14) ausbildet.

10. Medizinalröhrchen nach Anspruch 9, **dadurch gekennzeichnet, dass** der obere Behälterteil (30) und der untere Behälterteil (40) mittels umlaufenden Rastmitteln (31, 41), insbesondere einer umlaufende Wulst (42) und einer umlaufende Nut (32), miteinander in Eingriff bringbar sind.

11. Medizinalröhrchen nach einem der Ansprüche 9 bis 10, **dadurch gekennzeichnet, dass** zwischen dem oberen Behälterteil (30) und dem unteren Behälterteil (40) eine Verdrehsicherung (33, 43) angeordnet ist, vorzugsweise in Form mindestens einer Aussparung (44) und einem dazu komplementären Vorsprung (34).

12. Medizinalröhrchen nach einem der Ansprüche 9 bis 11, **dadurch gekennzeichnet, dass** der obere Behälterteil (30) den Probeentnahmekanal (10) ausbildet, welcher in den Behälterinnenraum (7) hineinreicht.

13. Medizinalröhrchen nach einem der Ansprüche 9 bis 12, **dadurch gekennzeichnet, dass** der Probeentnahmekanal (10) zweiteilig ausgebildet ist, wobei ein oberer, erster Kanalteil (15) durch den oberen Behälterteil (30) ausgebildet ist und ein unterer, zweiter Kanalteil (16) durch ein in den Behälterinnenraum hineinreichendes Röhrchen ausgebildet ist, welches fest im oberen Behälterteil (30) gehalten ist.

14. Medizinalröhrchen nach Anspruch 13, **dadurch gekennzeichnet, dass** Innenflächen des oberen ersten Kanalteils (15) im Übergangsbereich zum unteren, zweiten Kanalteil (16) mit den Innenflächen des unteren, zweiten Kanalteils (16) bündig abschliessen.

15. Medizinalröhrchen nach einem der vorangehenden Ansprüche, **dadurch gekennzeichnet, dass** die obere Probeentnahmeöffnung (11) im oberen Bereich des Medizinalröhrchens (1), vorzugsweise im oberen Viertel der Behälterwand (3) oder direkt unterhalb des Behälterdeckels (6), angeordnet ist.

## Claims

1. Medicinal tube for processing a biological sample by means of density gradient centrifugation and/or passive sedimentation, comprising a tube-shaped container (1) with an essentially cylindrical container wall (3), an upper filling opening (4) and a lower container base (5) and, covering the upper filling opening (4), a container cap (6), more particularly a screw-type cap; wherein the container well (3) encloses an interior space (7) of the container; wherein arranged in the container (1) is a sample removal channel (10) which has an upper sample removal opening (11) and lower sample removal opening (12), wherein the sample removal (10) opens out through the lower sample opening (12), in the area of the container base (5), into the container inner space (7), **characterised in that** the upper sample removing opening (11) for removing a sample by means of a sample removing tube, is arranged laterally in the upper area in the container wall (3) so the when the container cap (6) is closed, a sample can be removed through the upper sample removal opening (11), whereby for easier introduction of the sample removal tube, a turn-around area (13) of the sample removal channel (10) adjoining the sample removal opening (11) has a rounded, upper turn-around surface (14).

2. Medicinal tube according to claim 1, **characterised in that** the container comprises a closing element (20) covering the upper sample removal opening (11) .

3. Medicinal tube according to claim 2, **characterised in that** the closure element is an opening slider (20) for opening and closing the upper sample removal opening (11).

4. Medicinal tube according to claim 3, **characterised in that** the opening slider (20) is a sleeve (21) which encompasses the container (1), and has an opening (22), which, for opening the upper sample removal opening, (11) can be overlapped therewith.

5. Medicinal tube according to any one of claims 3 or 4, **characterised in that** the opening slider (20) and the container (1) are each provided with a stop (23, 38), which are in contact with each other when the opening slider is fully open.

6. Medicinal tube according to any one of claims 3 to 5, **characterised in that** the opening slider (20) and the container (1) have locking means (25, 39) which lock the opening slider in an open position and in closed position.

7. Medicinal tube according to claim 2, **characterised in that** the closing element is a tear-off sleeve covering the sample removal opening, which in order to open the sample removal opening, can be at least partially torn off and along a tearing seam has predetermined breaking point or predetermined tearing points.

8. Medical tube according to any one of claims 2 to 6, **characterised in that** the closing element (20) is recessed into the container wall (2) so that the outer surfaces of the container wall (2) and the closing element (20) are flush with each other at least below the closing element.

9. Medicinal tube according to any one of the preceding claims, **characterised in that** the container (1) is designed in two parts of an upper container part (30) and a lower container part (40), wherein the upper container part (30) and the lower container part (40) can be brought into engagement with each other and the upper container part (30) forms the sample removal opening (11) and the upper turn-around area (14).

10. Medicinal tube according to claim 9, **characterised in that** the upper container part (30) and the lower container part (40) can be brought into engagement with each other by means of circumferential locking means (31, 41), more particularly a circumferential bead (42) and a circumferential groove (32).

11. Medicinal tube according to any one of claims 9 to 10, **characterised in that** arranged between the upper container part (30) and the lower container part (40) is an anti-twist lock (33, 43), preferably in the form of at least one recess (44) and complementary projection (34).

12. Medicinal tube according to any one of claims 9 to 11, **characterised in that** the upper container part (30) forms the sample removal channel (10) which extends into the interior space (7) of the container.

13. Medicinal tube according to any one of claims 9 to 12, **characterised in that** the sample removal channel (10) is designed in two parts, wherein an upper, first channel part (15) is formed by the upper container part (30), and a lower, second channel part (16) is formed by a tube which is firmly held in the upper container part (30) and extends into the interior space of the container.

14. Medicinal tube according to claim 13, **characterised in that** in the transition area to the lower, second channel part (16), inner surfaces of the upper, first channel part (15) are flush with the inner surfaces of the lower, second channel part (16).

15. Medicinal tube according to any one of the preceding claims, **characterised in that** the upper sample removal opening (11) is arranged in the upper area of the medicinal tube (1), preferably in the upper quarter of the container wall (3), or directly below the tube cap (6).

## Revendications

1. Canule médicale pour préparer un échantillon biologique par centrifugation par gradient de concentration et/ou sédimentation passive comprenant un contenant (1) en forme de canule avec une paroi de contenant (3) essentiellement cylindrique, une ouverture de remplissage supérieure (4) et un fond de contenant inférieur (5), et un couvercle de contenant (6) recouvrant l'ouverture de remplissage supérieure (4), en particulier un couvercle vissé ; dans laquelle la paroi de contenant (3) entoure un intérieur de contenant (7) ; dans laquelle un canal de prélèvement d'échantillon (10) est disposé dans le contenant (1), lequel présente une ouverture de prélèvement d'échantillon supérieure (11) et une ouverture de prélèvement d'échantillon inférieure (12), dans laquelle le canal de prélèvement d'échantillon (10) débouche dans l'intérieur de contenant (7), au niveau du fond de contenant (5), à travers l'ouverture de prélèvement d'échantillon inférieure (12), **caractérisée en ce que** l'ouverture de prélèvement d'échantillon supérieure (11) est disposée latéralement dans la partie supérieure dans la paroi de contenant (3) pour prélever un échantillon au moyen d'un tube de prélèvement d'échantillon, de façon à ce que l'échantillon puisse être prélevé à travers l'ouverture de prélèvement d'échantillon supérieure (11) lorsque le couvercle de contenant (6) est fermé, dans laquelle une zone de déviation (13) du canal de prélèvement d'échantillon (10) se raccordant à l'ouverture de prélèvement d'échantillon supérieure (11) présente une face supérieure de déviation arrondie (14) pour une introduction plus facile du tube de prélèvement d'échantillon.

2. Canule médicale selon la revendication 1, **caractérisée en ce que** le contenant présente un élément de fermeture (20) recouvrant l'ouverture de prélèvement d'échantillon supérieure (11).

3. Canule médicale selon la revendication 2, **caractérisée en ce que** l'élément de fermeture est un tiroir d'ouverture (20) pour ouvrir et fermer l'ouverture de prélèvement d'échantillon supérieure (11) .

4. Canule médicale selon la revendication 3, **caractérisée en ce que** le tiroir d'ouverture (20) est un manchon (21) entourant le contenant (1), lequel présente une ouverture (22) qui peut être amenée en recouvrement avec celui-ci pour ouvrir l'ouverture de prélèvement d'échantillon supérieure (11) .

5. Canule médicale selon l'une des revendications 3 ou 4, **caractérisée en ce que** le tiroir d'ouverture (20) et le contenant (1) sont dotés chacun d'une butée (23, 38), qui sont en butée l'une avec l'autre lorsque le tiroir d'ouverture est complètement ouvert.

6. Canule médicale selon l'une des revendications 3 à 5, **caractérisée en ce que** le tiroir d'ouverture (20) et le contenant (1) présentent des moyens d'encliquetage (25, 39) qui encliquètent le tiroir d'ouverture dans une position ouverte et dans une position fermée.

7. Canule médicale selon la revendication 2, **caractérisée en ce que** l'élément de fermeture présente un manchon à arracher recouvrant l'ouverture de prélèvement d'échantillon qui peut être au moins partiellement arraché pour ouvrir l'ouverture de prélèvement d'échantillon et présente des points de rupture de consigne ou des points d'arrachement de consigne le long d'une couture à déchirer.

8. Canule médicale selon l'une des revendications 2 à 6, **caractérisée en ce que** l'élément de fermeture (20) est engagé dans la paroi de contenant (2) de telle façon que des parois extérieures de la paroi de contenant (2) et de l'élément de fermeture (20) affleurent l'une avec l'autre au moins en-dessous de l'élément de fermeture.

9. Canule médicale selon l'une des revendications précédentes, **caractérisée en ce que** le contenant (1) est constitué de deux parties, d'une partie de contenant supérieure (30) et d'une partie de contenant inférieure (40), dans laquelle la partie de contenant supérieure (30) et la partie de contenant inférieure (40) peuvent être mises en prise l'une avec l'autre, et la partie de contenant supérieure (30) forme l'ouverture de prélèvement d'échantillon supérieure (11) et la face de déviation supérieure (14).

10. Canule médicale selon la revendication 9, **caractérisée en ce que** la partie de contenant supérieure (30) et la partie de contenant inférieure (40) peuvent être mises en prise l'une avec l'autre par des moyens d'encliquetage périphériques (31, 41), en particulier un boudin périphérique (42) et une rainure périphérique (32).

11. Canule médicale selon l'une des revendications 9 à 10, **caractérisée en ce qu'**une sécurité anti-rotation (33, 43) est disposée entre la partie de contenant supérieure (30) et la partie de contenant inférieure (40), de préférence sous forme d'au moins un évidement (44) et d'une saillie (34) complémentaire à celle-ci.

12. Canule médicale selon l'une des revendications 9 à 11, **caractérisée en ce que** la partie de contenant supérieure (30) forme le canal de prélèvement d'échantillon (10) qui va jusque dans l'intérieur de contenant (7).

13. Canule médicale selon l'une des revendications 9 à 12, **caractérisée en ce que** le canal de prélèvement d'échantillon (10) est formé en deux parties, dans laquelle une première partie de canal supérieure (15) est formée par la partie de contenant supérieure (30), et une seconde partie de canal inférieure (16) est formée par une canule allant jusque dans l'intérieur de contenant, laquelle est maintenue fixement dans la partie de contenant supérieure (30).

14. Canule médicale selon la revendication 13, **caractérisée en ce que** des faces intérieures de la première partie de canal supérieure (15) terminent à l'affleur avec les faces intérieures de la seconde partie de canal inférieure (16) dans la partie de jonction vers la seconde partie de canal inférieure (16) .

15. Canule médicale selon l'une des revendications précédentes, **caractérisée en ce que** l'ouverture de prélèvement d'échantillon supérieure (11) est disposée dans la partie supérieure de la canule médicale (1), de préférence dans le quart supérieur de la paroi de contenant (3) ou directement en-dessous du couvercle de contenant (6).
